(19) Europäisches Patentamt
European Patent Office
Office européen des brevets

(11) Publication number: **0 030 838**
**B1**

(12) # EUROPEAN PATENT SPECIFICATION

(45) Date of publication of patent specification: **07.03.84**

(51) Int. Cl.³: **A 61 F 1/00**, C 08 L 83/04

(21) Application number: **80304431.2**

(22) Date of filing: **09.12.80**

(54) **Silicone gel-filled silicone rubber article possessing reduced surface-bleed.**

(30) Priority: **17.12.79 US 104480**

(43) Date of publication of application:
**24.06.81 Bulletin 81/25**

(45) Publication of the grant of the patent:
**07.03.84 Bulletin 84/10**

(84) Designated Contracting States:
**BE IT**

(56) References cited:
**WO - A - 80/02107**
**DE - B - 1 669 883**
**DE - B - 2 711 383**
**US - A - 3 366 975**
**US - A - 3 559 214**
**US - A - 3 683 424**
**US - A - 3 688 318**

(73) Proprietor: **DOW CORNING CORPORATION**
**Midland Michigan 48640 (US)**

(72) Inventor: **Larson, Willard Dale**
**1957 Richal Drive**
**Woodbury Minnesota 55119 (US)**

(74) Representative: **Lewin, John Harvey**
**Elkington and Fife High Holborn House 52/54**
**High Holborn**
**London WC1V 6SH (GB)**

**EP 0 030 838 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Silicone gel-filled silicone rubber article possessing reduced surface-bleed

This invention relates to silicone gel-filled silicone rubber articles.

The combination of a flexible silicone rubber container with a silicone gel composition is known and such articles are presently used in the field of medicine as external mammary prostheses or as surgically implantable prostheses for humans. In U.S. Patent No. 3,293,663, issued December 27, 1966 Cronin describes a surgically implantable mammary prosthesis which is basically a flexible silicon rubber container filled with a silicone gel which can be a gel of the type taught by Nelson in U.S. Patent No. 3,020,260 issued February 6, 1962. Other silicone gel-filled surgically implantable prostheses possessing silicone rubber containers are taught in U.S. Patent Nos. 3,665,520 issued May 30, 1972 to Perras et al, and 3,902,198 issued September 2, 1975 to Rathjen while similar prostheses designed for external use are described in U.S. Patent Nos. 3,896,506 issued July 29, 1975 to Hankin et al. and 3,911,503 issued October 14, 1975 to Hankin. The above patents describe methods for the manufacture, design and use of both implantable and external prostheses. The combination of a silicone rubber container with a silicone gel composition is desirable due to the low reactivity of the body towards silicone materials and because of the ease with which silicone materials can be cleaned and sterilized by steam or boiling water.

As noted in U.S. Patent No. 4,100,627 issued July 18, 1978 to Alfred P. Brill, III, a problem exists when a silicone rubber container is filled with a silicone gel composition. There is a tendency for some of the components of the silicone gel composition to exude or bleed through the container wall and to cause the surface of the article to feel oily or greasy. In the case of an external prosthesis, the patient is likely to object to the oily feel or the exudate will stain the patients' clothing and result in embarrassment. This problem is especially noticeable when both the container and the gel composition are derived from polydimethylsiloxanes. Thus, it is desirable to reduce or eliminate the exudation, oiling or bleed of the gel components through the silicone rubber container. When the exception of Brill the above patents do not appear to address the bleed problem and only state in general terms that the container walls be "impervious" to the materials contained therein while Cronin teaches that the "gelled material should be one which is inert toward the container" (col. 3, lines 1—2).

Brill teaches that the prior art silicone gel-filled silicone rubber containers have an undesirable amount of oiling or bleed because in order to obtain the desired penetration values for the gel with the appropriate natural simulated characteristics, large amounts of unreacted fluid were included in the gel network. Components present in these fluids can then bleed through the silicone rubber container walls to cause the above problem. Brill's solution to the bleed problem resides in utilizing a particular type of silicone gel as a filling material. However, a great deal of effort has been made in years past to develop silicone gel compositions with the proper characteristics and many silicone gell compositions are commercially available. Proper characteristics, such as penetration values, are extremely important when the article is to be used as a mammary prosthesis. For example, if the external mammary prosthesis does not feel natural to the touch, the potential wearer is likely to reject it for purely aesthetic reasons regardless of the amount of surface bleed which the prosthesis may possess. Thus, it is desirable to produce a silicone gel-filled silicone rubber article possessing reduced surface-bleed which is capable of utilizing various types of silicone gel compositions, such as those mentioned in Cronin and taught by Nelson, which possess the required characteristics for the articles' intended use.

Barrier membranes used in implantable prostheses are taught by Pangman in U.S. Patent No. 3,366,975 issued February 6, 1968. Pangman teaches a prosthesis which consists of (a) a foam type plastic or silicone sponge core which can also be filled with a silicone gel, (b) a barrier membrane which is typically a silicone polymer, surrounding and bound to the core which is impervious to cellular tissue and fluids and (c) an open cell foam layer covering the membrane which can be invaded by body tissue. A similar type of prosthesis provided with a means for changing the quantity of fluid inside the core is described by Pangman in U.S. Patent No. 3,683,424 issued August 15, 1972. The above patents to Pangman neither address the problem nor do they suggest a means for reducing the surface-bleed of an article comprising a silicone gel-filled silicone rubber container.

In a data sheet entitled "Natrashiel™ Mammary Implant" (McGhan Medical Corporation, Santa Barbara, CA 93111—Sheet No. 120388, 10/78), a gel-filled mammary implant which consists of a conventional silica-containing silicone elastomer implant shell containing an outer coating of a filler-free silicone polymer is described. The stated purpose for the outer coating is "to provide a shield barrier between body tissue, and the silica containing silicone elastomer implant shell" (Data Sheet, p. 1). No mention is made concerning the problem of surface-bleed when the gel is a silicone gel.

It is thus apparent that in the past, the problem of reducing the surface-bleed of silicone gel-filled silicone rubber articles has either not been addressed or when it has, the

solution proposed still has room for improvement. A combination of materials has been discovered which provides a novel solution to the above problem.

It is a principal object of this invention to present a solution to the above-described bleed problem.

It is also an object of the present invention to provide a silicone gel-filled silicone rubber article possessing a reduced tendency to exhibit surface-bleed.

It is another object of the present invention to provide a silicone gel-filled silicone rubber article possessing reduced surface-bleed which is useful as an externally worn mammary prosthesis.

It is still another object of the present invention to provide a silicone gel-filled silicone rubber article possessing reduced surface-bleed which is useful as a surgically implantable prosthesis such as a testicular implant, pillow or pad, and especially as a prosthesis to be used as a mammary implant.

These and other objects are accomplished by a silicone gel-filled silicone rubber article possessing a reduced tendency to exhibit surface-bleed comprising (A) a flexible silicone rubber container filled with (B) a silicone gel composition, there being (C) an essentially continuous barrier layer of a composition consisting essentially of a fluorine-containing organopolysiloxane situated between the interior of container (A) and the gel of (B). The composition (C) contains a sufficient amount of a fluorine-containing organopolysiloxane which is sufficiently incompatible with the components found in gel (B) to retard the passage of migratable gel components into the container (A) and thereby reduce the amount of bleed appearing on the surface of (A).

Alternatively, the invention relates to a flexible silicone gel-filled silicone rubber article possessing a reduced tendency to exhibit surface-bleed consisting essentially of a flexible fluorosilicone rubber container filled with a silicone gel composition.

In the accompanying drawings: Figure 1 is a perspective view of a surgically implantable prosthesis shaped to supplement a female breast, constructed in accordance with the teachings of the present invention, and

Figure 2 depicts a cross-section on a vertical median of the prosthesis of Figure 1.

The present invention relates to a silicone gel-filled silicone rubber article possessing a reduced tendency to exhibit surface-bleed comprising:

(A) a flexible container of silicone rubber consisting essentially of a cross-linked gum of polydimethylsiloxane, said container being filled with
(B) a silicone gel composition consisting essentially of a silicone gel of cross-linked polydimethylsiloxane, there being
(C) an essentially continuous barrier layer of a composition consisting essentially of a fluorine containing organopolysiloxane situated between the interior of container (A) and the gel composition of (B), said barrier layer being coated on the interior of container (A).

The present invention also relates to a silicone gel-filled silicone rubber article possessing a reduced tendency to exhibit surface-bleed consisting essentially of

(A) a flexible container of fluorosilicone rubber consisting essentially of a cross-linked gum of fluoroalkylpolysiloxane filled with
(B) a silicone gel composition consisting essentially of a silicone gel of cross-linked polydimethylsiloxane.

Referring to the drawings, Figure 1 shows one embodiment of the present invention shown as P which is a surgically implantable prosthesis shaped to replace a female breast and constructed in accordance with the present invention. The outside container is shown as 21. Other types of embodiments will be readily apparent to those skilled in the art because the basic construction can be altered to assume the particular shape required. For example, an external breast prosthesis designed for use within a brassier cup would have a similar shape. The actual shape of the article does not form a part of the present invention and the shape shown is solely for the purpose of illustration.

From Figure 2, it will be apparent that the article of the present invention comprises three main parts: a flexible silicone rubber container 21, a silicone gel composition filler material 23 located within the container and an essentially continuous barrier layer 22 situated between container 21 and gel composition 23. The princpal requirement of the present invention is that the barrier layer 22 be an essentially continuous coating covering the entire inside wall area of container 21. Uniformity of coating thickness is generally not critical as long as the coating is essentially continuous. The term "essentially" continuous is meant to indicate that the presence of a small amount of pin-holes or other small areas of surface coating defects can be tolerated, although it is preferred that no such defects be present.

The silicone rubber container 21 is based on a gum which has been cross-linked or to use other commonly accepted terms, "vulcanized" or "cured". The gum is principally composed of a polydimethylsiloxane. By the term "polydimethylsiloxane", it is to be understood that the majority of the siloxane units present in the gum are dimethylsiloxane units, but that minor amounts of other diorganosiloxane units can be present such as methylvinylsiloxane units, methylphenylsiloxane units and diphenylsiloxane units. The gum can also contain minor

amounts of monoorganosiloxane units and $SiO_2$ units which provide branching. The gums can be endblocked by conventional endblocking units such as triorganosiloxy units such as dimethylvinylsiloxy units, trimethylsiloxy units or methylphenylvinylsiloxy units or by hydroxyl radicals. Since these gums are well-known to those skilled in the art, no further elaboration is necessary.

It should be noted that the tendency for silicone gel-filled silicone rubber articles to exhibit surface-bleed is dependent upon the composition of the gel and the composition of the container. For example, assume that the silicone rubber container is essentially composed of a cross-linked polydimethylsiloxane and the silicone gel composition is essentially composed of polydimethylsiloxane components. If the gel composition is kept the same and methylphenylsiloxane units are substituted for a portion of the dimethylsiloxane siloxane units in the silicone rubber container, as the mole percentage of methylphenylsiloxane units is increased, the tendency for the unreacted components present in the gel composition to bleed through the container walls will decrease, although the difference may not be apparent until a large percentage of the container is composed of methylphenylsiloxane units. The same is true where the container material is kept constant and the silicone gel composition is varied as above. However, it is generally preferred to use silicone rubber containers formed from gums which are essentially composed of polydimethylsiloxane.

In an alternative embodiment, the flexible container 21 of Figure 2 can be manufactured from a fluorosilicone rubber consisting essentially of fluoroalkylpolysiloxane in which case there is no need for the presence of a barrier layer 22 in the article because the container itself can function as the barrier layer. The fluoroalkylpolysiloxanes are described infra and the preferred gum is 3,3,3-trifluoropropylmethylpolysiloxane. As described above, the fluorosilicone rubber can also contain fillers, additives and the like.

Silicone rubbers are well known and can contain fillers, such as reinforcing silica filler; processing aids, additives, and pigments such as titanium dioxide. The silicone rubber can be cross-linked or vulcanized by conventional means such as with organic peroxides, electromagnetic radiation or by using a polysiloxane cross-linker containing silicon-bonded hydrogen atoms with a vinyl-containing gum and a platinum catalyst. The proportions of these ingredients are well known in the art and those skilled in the silicone rubber art can readily adjust the ingredients and proportions to provide a silicone rubber which will suit their particular desires in physical properties for the container. For example, where an article is to be used as a mammary prosthesis, the container wall should be sufficiently soft and flexible so as to simulate a natural appearance and feel. Typically such containers are about 0.25 mm in average thickness.

The silicone gel filling material, shown as 23 in Figure 2, is a composition which consists essentially of a silicone gel of cross-linked polydimethylsiloxane, which composition can also include unreacted polyorganosiloxane fluids, additives, pigments, processing aids and the like. By the term "cross-linked polydimethylsiloxane", it is to be understood that the majority of the siloxane units present in the cross-linked gel portion of the gel composition are dimethylsiloxane units although lesser amounts of other diorganosiloxane units such as methylphenylsiloxane units may also be present. Silicone gel compositions suitable for use in the articles of the present invention are well known in the art. Silicone gel compositions are described by Cronin in U.S. Patent No. 3,293,663 issued December 27, 1966 (hereinafter referred to as Cronin), which refers to a specific type of the gels described by Nelson in U.S. Patent No. 3,020,260 issued February 6, 1962 (hereinafter referred to as Nelson). Brill, in U.S. Patent No. 4,100,627 issued July 18, 1978 describes another type of silicone gel. The Cronin, Nelson and Brill patents are hereby incorporated by reference to teach various types of silicone gel compositions which can be used in the articles of the present invention, methods for the production of such gels, penetration values useful for mammary prostheses and methods for determing the penetration values of such gels. While Cronin and Brill suggest gel penetration values which are desirable for use in mammary prostheses, it is well known in the art that other penetration values can be obtained by suitable modification of the silicone gel composition such as by the addition of nonreactive trimethylsiloxy-endblocked polydimethylsiloxane fluids or to alter the ratios of reactants when a softer or stiffer gel is required. To obtain maximum reduction in bleed due to the presence of the barrier layer, it is preferable that the gel and any additives containing siloxane units have methyl radicals as the majority of the substituents attached to silicon atoms by means of carbon-silicon bonds.

The barrier layer 22 forms the basis for the reduced tendency of the silicone gel-filled silicone rubber articles described herein to exhibit surface bleed. While not intending to limit the present invention by the following theoretical discussion, it is believed that some discussion of the reasoning behind the use of the barrier layer 22 will aid others in the utilization of these articles. The barrier layer 22 is an essentially continuous barrier layer of a preferably filler-containing composition comprising a fluorine-containing organopolysiloxane. Fluorine-containing organopolysiloxanes tend to be more or less chemically incompatible with organopolysiloxanes which do not contain fluorine such as polydimethylsiloxane fluids. The actual

amount of incompatibility is related to the substituents present in each type of polyorganosiloxane. It is believed that this incompatibility is responsible for the reduced surface-bleed exhibited by the articles of the present invention. Therefore, in formulating the barrier layer 22, one must keep in mind that the composition of the barrier layer must be such that it presents an incompatible layer of a sufficient thickness to contain the components of the silicone gel 23 which would normally tend to migrate through the silicone rubber container 21 in the absence of a barrier layer and as a result, render the surface of the container oily.

Fluorine-containing organopolysiloxanes useful in the barrier layer 22 of the present invention can be those which are sufficiently incompatible with the components of the silicone gel so as to inhibit their migration to the silicone container 21, are capable of being used in a composition which forms an essentially continuous barrier layer on the interior of container 21, and especially when utilized in surgically implantable prostheses, are sufficiently inert with respect to the human body to be useful in such articles.

The fluorine-containing organopolysiloxanes used in the barrier layer composition can be selected from fluoroalkylpolysiloxanes such as those described in U.S. Patent Nos. 2,961,425 issued November 22, 1960 to Pierce et al.; 3,002,951 issued October 3, 1961 to Johannson; 3,006,878 issued October 31, 1961 to Talcott; 3,179,619 issued April 20, 1965 to Brown and 3,697,473 issued October 10, 1972 to Polmanteer et al. which are referred to to show the types of fluoroalkylpolysiloxanes which can find use as the barrier layer 22 and to show methods for the preparation of such fluoroalkylpolysiloxanes. These fluoroalkylpolysiloxanes can be described as being principally composed of siloxane units of the formula

$$(RCH_2CH_2)R'SiO$$

where R is a perfluoroalkyl radical of less than 11 carbon atoms such as $CF_3$-, $C_2F_5$-, $C_3F_7$- and $C_{10}F_{21}$- and can be either straight chain or branched chain radicals while R' is an alkyl radical of 1 to 3 inclusive carbon atoms such as methyl, ethyl, propyl, or isopropyl. Hydroxyl-endblocked homopolymers consisting almost entirely of

$$(RCH_2CH_2)R'SiO$$

units (it being recognized that small amounts of mono-substituted, trisubstituted or $SiO_2$ siloxane units can be present due to byproducts present during manufacture) can be utilized. Polydiorganosiloxanes containing up to 10 mole percent, based upon the total moles of siloxane units present, of non-fluorine containing siloxane units of the average formula

$$R''_xSiO_{\frac{4-x}{2}}$$

wherein each R″ is selected from the group consisting of alkyl radicals of 1 to 2 inclusive carbon atoms such as methyl or ethyl, vinyl radicals and phenyl radicals and *x* has an average value of from 1 to 3 inclusive can also be used. The use of various types of

$$R''_xSiO_{\frac{4-x}{2}}$$

siloxane units enables one to select various means for cross-linking these fluoroalkylpolysiloxanes and examples of these siloxane units include the following units:

$(CH_3)(C_2H_4)SiO$,
$(CH_3)_2SiO$,
$(CH_3)(C_2H_4)(C_6H_5)SiO_{0.5}$,
$(CH_3)_3SiO_{0.5}$,
$CH_3SiO_{1.5}$ and
$(CH_3)(C_2H_5)_2SiO_{0.5}$.

Larger amounts of

$$R''_xSiO_{\frac{4-x}{2}}$$

units can be used in such copolymers, but the ability of a barrier layer to reduce bleed is expected to decrease as the amount of nonfluorine-containing siloxane units is increased beyond 10 mole percent.

Due to considerations such as cost and commercial availability, the preferred type of fluoroalkylpolysiloxane to use as the barrier layer 22 are those which are principally composed of

$$\{(CF_3CH_2CH_2)(CH_3)SiO\}$$

siloxane units, and contain less than 10 mole percent of

$$R''_xSiO_{\frac{4-x}{2}}$$

siloxane units where each R″ is a methyl or vinyl; radical, there being no more than one vinyl radical per siloxane unit (hereinafter referred to as 3,3,3-trifluoropropylmethylpolysiloxanes). More preferably, the 3,3,3-trifluoropropylmethylpolysiloxane is a gum which is capable of being cross-linked by means of a platinum catalyst method. A gum is generally defined to be a polydiorganosiloxane with a viscosity of over 1 meter$^2$ per second (1,000,000 centistokes) and is usually described in terms of its Williams plasticity which is described in ASTM D926.

It was also observed that the barrier-layer compositions exhibited poor coating or wet-out properties on the silicone rubber container in the absence of a filler. Low viscosity trimethylsiloxy-endblocked 3,3,3-trifluoropropylmethylpolysiloxane fluids having a viscosity of about 300 centistokes (0.3 Pa · s (pascal seconds)) and high molecular weight 3,3,3-trifluoropropylmethylpolysiloxane gum dispersions in acetone were evaluated by injecting the fluid or dispersion into a reinforcing silica filler-containing silicone rubber container consisting primarily of cross-linked polydimethylsiloxane and observing the coating formed on the inside of the container. It was found that when only extending fillers such as barium sulfate, diatomaceous earth, sodium bicarbonate or crystalline silica were used, more than 1 weight percent of extending filler based upon the total weight of the fluid was required to obtain a coating on the inside of the silicone rubber container which did not bead up or become discontinuous within five minutes after coating. With high viscosity gums, more than 0.5 weight percent of extending filler alone was required. More than 0.5 weight percent of filler was required when a reinforcing silica filler such as a surface-treated fume silica was evaluated. Combinations of various fillers can also be used. Many other extending and reinforcing fillers are known to those skilled in the art and need not be described further. In general, the coating uniformity increases with increasing amounts of filler. Routine evaluation of filler levels can be used to optimize the level of filler needed for a particular application. In some cases, it can be possible to uniformly coat the inside of a silicone rubber container with an acetone or methyl isobutyl ketone dispersion of a high molecular weight fluorine-containing polyorganosiloxane composition which does not contain any filler if the viscosity of the polyorganosiloxane or the coating dispersion is sufficiently high and if the dispersion is freshly prepared and used. However, it is preferable to use at least one filler to obtain consistent coatings and also to reduce the cost of the coating composition.

In addition to the gum and filler, the barrier layer composition can contain small amounts of various additives such as plasticizers, colorants, dyes or compounds and/or polymers used to cross-link or cure the flourine-containing organopolysiloxane. When a silicone rubber container is uniformly coated with a barrier layer composition which remains on the container walls and does not flow off during handling and the silicone gel is then introduced into the coated container, the barrier layer appears to reduce bleed even though the fluorine-containing organopolysiloxane has not been cross-linked. This observation appears to be due to the incompatibility of the silicone gel with the barrier layer composition. It is, however, preferred that the fluorine-containing organopolysiloxane be cross-linked to insure that the barrier layer remains uniformly coated over the inside of the entire container during the useful life of the article.

Cross-linking can be accomplished by any of a number of well-known means such as by peroxides, radiation, and silanol condensation reactions catalyzed by metal carboxylic acid salts. Room temperature vulcanization of fluorine-containing compositions is taught in U.S. Patent No. 3,624,022 issued November 30, 1971 to Ross (silane additive plus a metal salt of a carboxylic acid) and in U.S. Patent No. 3,077,465 issued February 12, 1963 to Bruner (moisture-curable acetoxy-endblocked polyorganosiloxane).

The preferred method for cross-linking the fluorine-containing organopolysiloxane in the barrier-layer composition is by means of a platinum-catalyzed reaction between siloxane units containing hydrogen bonded directly to a silicon atom and siloxane units containing unsaturated alkyl radicals bonded to silicon atoms by means of a carbon-silicon bond such as vinyl radicals. The means for accomplishing platinum catalyzed cross-linking of fluorine-containing polydiorganosiloxanes is set out in the literature such as in U.S. Patent No. 3,697,473 issued October 10, 1972 to Polmanteer, et al. and U.S. Patent No. 3,445,420 issued May 20, 1969 to Kookootsedes, et al. which are hereby incorporated by reference to teach: this method of cross-linking, the silicon-bonded hydrogen organosiloxane compounds used to effect cross-linking and the method of their preparation, the platinum catalysts which can be used and the amounts to be used and the types of inhibitors which can be used to inhibit the action of the platinum catalyst, if such cure inhibition is desired. Preferably, the platinum catalysts used are those described in U.S. Patent No. 3,419,593 and the preferred class of inhibitors is the acetylenic compounds set out in the Kookootsedes, et al. patent which patents are both hereby incorporated by reference to show the preparation of the preferred catalysts and inhibitors and to further describe each type of compound. Thus, methods for the formulation and cure of fluorine-containing polyorganosiloxanes are well known to those skilled in the art and do not need to be discussed in detail.

The barrier layer compositions are preferably applied as dispersions in polar organic solvents such as ketones such as acetone and methyl isobutyl ketone, esters or chlorinated hydrocarbons such as 1,1,1-trichloroethane. The solvent of choice for the dispersions containing 3,3,3-trifluoropropylmethylpolysiloxane gums was acetone. As noted earlier, when lower viscosity 3,3,3-trifluoropropylmethylpolysiloxane was used (viscosity approximately 3000 centipoise (3 Pa · s)) some coating problems were encountered when the container and barrier layer were produced by the dipping

process hereinafter described. However, when the silicone rubber containers are pre-formed and the barrier-layer composition is applied by injecting a dispersion of the same into the container as described infra, adequate barrier-layer coatings can be obtained upon curing such compositions.

The silicone gel-filled articles illustrated by Figures 1 and 2 can be manufactured by several methods. In the first method, the container 21 can be formed by conventional means such as by vacuum-forming or by dip-coating a mandrel of the desired size into a solution or dispersion of a composition which will then be cured such as by heating to form the flexible silicone rubber container. After curing, the container 21 is stripped from the mandrel leaving a small opening which can be sealed by means of a patch made from the same type of silicone rubber material as is the container and a drop of high strength silicone elastomer can be used to adhere the patch to the container. Alternatively, the patch itself can be unvulcanized and simply placed over the hole to be vulcanized or cured at a later time. The inside of the container is then coated by first inserting a hypodermic needle through the wall of the container and introducing a sufficient amount of air into the container to produce a wrinkle-free surface which can be evenly coated with the barrier-layer composition. The barrier-layer composition is then introduced through the hypodermic needle, typically as a solvent dispersion, the needle hole is sealed and the containers are tumbled in a rotating drum to evenly coat the interior of the container. Depending upon the type of cure system chosen for the barrier-layer composition, after a short period of tumbling to coat the container, heat can be introduced to form a cured barrier layer. The injected air and excess solvent generally escape during the tumbling and/or curing operation leaving the barrier-layer-containing silicone rubber containers ready for filling with silicone gel composition. Uncured silicone gel composition is then introduced by means of a hypodermic needle and the silicone gel is cured such as by heating the article. It may be possible to introduce a cured silicone gel directly into the container if the gel is capable of passing through the injecting means.

Another method for preparing the silicone gel-filled silicone rubber articles of the present invention, especially those useful as mammary prostheses, consists of using a conventional dip-coating technique which comprises first dip-coating a mandrel of the proper shape to form a coating of the uncured silicone rubber container material and then dip-coating that coated mandrel in a solution of dispersion of the uncured barrier-layer composition. This method is preferred over the previously described method when dispersions containing non-flammable solvents are used or where adequate provision for maintaining dipping tanks of flammable solvents is available because the process is more commercially efficient. The coatings on the mandrel are then cured to form a container with a cured barrier layer. The container is then stripped from the mandrel, leaving a small hole which is sealed. When the container is stripped from the mandrel, it is turned inside out resulting in a container having a barrier layer on the interior wall. The shape of the mandrel can be either circular or ellipsoidal depending upon the desired container shape while the size is dictated by the container volume desired. Such mandrels and procedures for their use are well known in the art. The uncured silicone gel composition is then injected and cured (or cured silicone gel may be injected) to form a silicone gel-filled silicone rubber article with a reduced tendency to exhibit surface-bleed.

While it is recognized that the barrier layer could comprise a reinforcing silica filled fluorine-containing organopolysiloxane composition coating on the outside of the silicone rubber container and some reduction in bleed could be observed, the preferred procedure is to use a barrier layer on the inside of the container wall to reduce the amount of gel components reaching the silicone rubber container.

It is of course understood by those skilled in the art that the silicone gel-filled silicone rubber article described above is directed towards an article with reduced tendency to exhibit surface-bleed and that the physical shape of the article can be altered without affecting the reduced-bleed characteristics provided that the barrier layer is essentially continuous. Likewise, various types of modifications to the outside of the gel-filled articles described herein may be made without affecting the reduced-bleed characteristics. For example, a layer of porous material can be attached to the outside of the article to enable body tissue to grow into such porous material and anchor the prosthesis to the chest wall (see Cronin cited above). In the case of externally worn articles such as external mammary prostheses, the articles can have appendages which are designed to properly fit a brassier and such prostheses can also contain pigments and colorants to produce a natural, flesh-tone appearance. These and other reasonable modifications to the articles described herein will be apparent to those skilled in the art and are felt to be within the scope of the invention described in the claims below.

The preferred embodiment of the present invention is a silicone gel-filled silicone rubber article with a reduced tendency to exhibit surface-bleed and is shaped in the form of either an external mammary prosthesis or a surgically implantable article, especially one which is useful as an implantable mammary prosthesis. These preferred prostheses comprise a flexible silicone rubber container 21 which consists essentially of a cross-linked gum essentially

composed of polydimethylsiloxane; a silicone gel composition 23 filling material which consists essentially of a gel which is a cross-linked essentially polydimethylsiloxane wherein any additives present in the gel which contain siloxane units have methyl radicals as the majority of the substituents attached to silicon atoms by means of carbon-silicon bonds and a barrier layer 22 which is a filler-containing composition consisting essentially of a cross-linked gum consisting essentially of 3,3,3-trifluoropropylmethylsiloxane. In a more preferred embodiment, the container 21, the silicone gel 23 and the barrier layer 22 are each cross-linked by means of a platinum catalyst to avoid the possibility of platinum catalyst poisoning due to the presence of residual amounts of another type of curing agent, such as a peroxide, which could be present if, for example, the container was peroxide-cured.

Several methods for evaluating the bleed of silicone gel-filled articles were used. Method I involved preparing the gel-filled articles, cleansing the surface with isopropanol and drying the articles to be tested. Each sample was placed in a circular covered glass tissue culture dish (60 mm in diameter by 15 mm high which are sometimes referred to as "Petri" dishes) between two pieces of white laboratory filter paper 5.5 centimeters (cm) in diameter. The sample containing dishes were stacked in layers between 10 cm×15 cm×0.16 cm aluminum plates—four dishes per layer with the top layer having three dishes and a weight at the very top— and the stack was stored at ambient temperature. At various periods of time, the samples were withdrawn, the paper was examined for the presence of spotting due to bleed and the condition of the containers was visually observed and evaluated. After two days time, blue writing paper was substituted for the white laboratory filter paper to make the presence of spots due to bleed more readily visible.

Method II involved weighing the paper on which the silicone gel-filled article was to be stored with an analytical balance and recording the increase in weight of the paper as a function of time, said increase being attributed to material exuding or bleeding through the container walls.

The following examples are intended as being merely illustrative and are not to be construed as limiting the scope of the present invention, which is properly defined by the appended claims. All parts and percentages are by weight unless otherwise noted.

Example 1—9

The following examples demonstrate the method used in preparing the articles of the present invention utilizing a dip-coating procedure. The following dispersions were prepared:

Dispersion A was prepared using a polydimethylsiloxane gum base (Base 1) which was a mixture of a dimethylvinylsiloxy-endblocked polydiorganosiloxane gum containing dimethylsiloxane units and about 0.14 mole percent of methylvinylsiloxane units based on the total moles of siloxane units present and having a plasticity in the range of 55—65 mils (1.40—1.65 mm) and a surface-treated fume silica filler having an average pore volume of about 400 $m^2/g$ prior to surface modification in the presence of the gum with hexamethyldisilazine in the presence of water wherein the weight ratio of gum to treated silica filler was about 63/37 and the base further contained 0.25 weight percent of zinc stearate. The plasticity reported in these examples is the thickness of the sample after being subjected to a force of 49 N for 3 minutes at 23±1°C using a plastometer of the type described in ASTM D926. Fifteen parts of Base 1 was dispersed in 85 parts of 1,1,1-trichloroethane to produce a dispersion. Then 0.04 parts of 2-methyl-3-butyn-2-ol was added to 100 parts of the above dispersion which was then stirred for at least one hour, and stored for at least 16 hours. After storing, 0.09 parts of Polymer 1 was added and dispersed by mixing for one hour. Polymer 1 was a trimethylsiloxy-endblocked polyorganosiloxane having 37.5 mole percent dimethylsiloxane units and 62.5 mole percent methylhydrogensiloxane units where the mole percent is exclusive of the trimethylsiloxy units and having a silicon-bonded hydrogen atom content in the range of 0.7 to 0.8 weight percent. Finally, prior to use, 0.09 parts of a soluble chloroplatinic acid catalyst (Catalyst 1) containing about 0.2 weight percent platinum metal content was added to form Dispersion A.

Dispersion B was a 10% solids dispersion prepared by adding 100 grams of Dispersion A to 40.3 grams of 1,1,1-trichloroethane and mixing well.

Dispersion C was prepared using the following gum base (Base 2) which consisted of 100 parts of a hydroxy-endblocked polydiorganosiloxane gum which contained 3,3,3-trifluoropropylmethylsiloxane units and about 0.6 mole percent of methylvinylsiloxane units and had a plasticity of 137 mils (3.48 mm), 1.9 parts of a hydroxy-endblocked polydiorganosiloxane gum which contained dimethylsiloxane units and about 4 mole percent of methylvinylsiloxane units and had a plasticity in the range of 50—70 mils (1.27—1.78 mm), 6 parts of a hydroxy-endblocked polydimethylsiloxane fluid having about 4 weight percent silicon-bonded hydroxyl radicals and having a viscosity of about $4\times10^{-5}$ $meters^2$ per second ($m^2/s$) at 25°C, and 21 parts of a reinforcing fume silica filler having a pore volume surface area of about 250 $m^2/g$. The base was prepared using the following general procedure. The first three ingredients were blended in a dough-type mixer, then the silica was added with stiring. The ingredients were heated for about 3 hours at 170°—180°C

under vacuum, cooled and strained to produce the base.

Eighty grams of acetone was added to 20 grams of Base 2 in a round jar and were thoroughly mixed by rolling the container on a roller mill overnight. Then 0.15 grams of a chloroplatinic acid complex (Catalyst 2) of divinyltetramethyldisiloxane diluted with a sufficient amount of a methylphenylvinylsiloxy-endblocked polydimethylsiloxane having a viscosity in the range of 0.3 to 0.5 Pa s and an average ratio of organic radicals per silicon atom in the range of 2.012 to 2.016 and 0.08 grams of 2-methyl-3-butyn-2-ol was added to the above dispersion and mixed by shaking and then rolling on a roller mill for one hour. The resulting dispersion was allowed to stand for 5 days before adding Polymer 2, described below, although it could have been added after the rolling was finished.

Before using the dispersion, 0.30 grams of Polymer 2 was added to the dispersion of the other ingredients and incorporated by shaking. Polymer 2 was a trimethylsiloxy-endblocked polyorganosiloxane having about 46 mole percent of 3,3,3-trifluoropropylmethylsiloxane units and about 54 mole percent of methylhydrogensiloxane units where the mole percent is exclusive of the trimethylsiloxy units and having a silicon-bonded hydrogen atom content in the range of 0.4 to 0.5 weight percent. Dispersion C contained about 20% solids.

Dispersion D contained about 11% solids and was prepared in the same manner as was Dispersion C using 80 grams of acetone, 10 grams of Base 2, 0.08 grams of Catalyst 2, 0.04 grams of 2-methyl-3-butyn-2-ol and 0.15 grams of Polymer 2.

A stainless steel youth size testicular envelope mandrel 2.4 cm×3.4 cm long which was capable of producing a container with a fill volume of 9.4 cubic centimeters was used to produce the containers used in these examples. The mandrel was attached to a 0.48 cm rod to produce a handle for dipping the mandrel. The rod was then attached to an electric motor drive dipping apparatus which was adjusted to allow the mandrel to be raised or lowered at a constant rate.

The mandrel was cleaned with solvent and slowly inserted into a container filled with the dispersion being used. The mandrel was then removed at the rate of 2 inches per minute. As soon as the mandrel was free from the surface, the mandrel was inverted and allowed to dry with the handle side down for at least five minutes time between dips. After the final coating was applied, the coated mandrel was allowed to dry for at least 10 minutes prior to placing it in an oven in an inverted position. Table I details the number of dips and the dispersions used in each dip. The following heating schedule was used to cure the coatings on the mandrels: Example 1—60 minutes at 150°C; Examples 2 and 3—60 minutes at 175°C and Examples 4—9—30 minutes at 175°C.

After curing, the mandrel was cooled under a tap water stream and dried. A standard No. 3 size cork borer was slipped over the handle to cut a hole through the top of the container. The cured container was then dusted with sodium bicarbonate to facilitate removal from the mandrel which removal was accomplished by rolling the envelope off the mandrel starting at the hole cut through the top. Thus, the container was turned inside out when the container was removed from the mandrel and the last layer to be coated became the inner layer. The thickness of the top (where the hole was made), the bottom and an average thickness was measured and is shown in Table I as is the composition of the envelope in number of dips starting from the inside layer outward.

The hole-patching procedure used in this bleed study was to impregnate 100 parts of Base 2 with 3 parts of a 50% active paste of 2,4-dichlorobenzoyl peroxide (Cadox® TS-50, a product of the Noury Chemical Company, Burt, NY 14028) on a roller mill, press a sample between two pieces of polyethylene coated paper to produce a sheet of unvulcanized gum base, remove one sheet of paper and place a piece of polyethylene terephthalate fabric between the gum base and the paper. The sheet was placed in a hydraulic press for about 15 minutes at a pressure of greater than $1.6\times10^8$ pascals to produce a sheet of about 0.38—0.51 mm in thickness. The containers were then washed in hot tap water and dried in an oven for 15 minutes at 150 C. A patch was cut from above using a standard size No. 7 cork borer and the paper was removed from the side opposite the fabric. This type of patch was then placed over the outside of the hole in each envelope except for Example 1, which was covered with a patch made from an unvulcanized polydimethylsiloxane gum base, and the patched envelopes were placed in an oven for 15 minutes at 150°C, to vulcanize the patch material. All the patches had good adhesion to the envelopes.

When a container with an outside surface which is completely composed of cross-linked polydimethylsiloxane is desired, a double layer patch can be used. A patch composed of an unvulcanized fluorine-containing polyorganosiloxane gum base which is slightly larger than the hole can be centered on a slightly larger patch composed of an unvulcanized polydimethylsiloxane gum base and the double-layered patch is then placed over the outside of the hole. It is readily apparent that the hole can also be patched from the inside by reversing the order of the layers such that the silicone gel-containing composition is only in contact with the barrier layer portion of the patch.

The patched containers were each filled with 8.00±0.25 grams of an uncured silicone gel composition by means of a 15 gauge hypodermic needle inserted directly through the container wall. The filled envelopes were washed with isopropanol after filling, air bubbles were removed and the filled containers were placed in an oven for three and one-half hours at 160°C to form the silicone gel. The cured silicone gel composition consisted of a uniform composition suitable for use in a mammary prosthesis containing about 80% by weight of unreacted trimethylsiloxy-endblocked polydimethylsiloxane fluid having a viscosity in the range of 0.00095 to 0.00105 $m^2/s$ at 25°C and about 20% of a cross-linked silicone gel consisting essentially of a polydimethylsiloxane of the type described in the Nelson Patent. The cured gel composition had a penetration value of 30.0 mm. The penetration value was determined according to the procedure taught in the Cronin Patent. Because the gel composition was rather soft, the following head and rod was used in place of the brass head and shaft described in Cronin—Head—an aluminum shaft about 15 cm long by 0.31 cm in diameter which was fitted with an aluminum tip 0.31 cm long and 2.54±0.02 cm in diameter (weight 7.5 g) and rod—a magnesium rod 21.6 cm long by 0.48 cm in diameter (weight 7.1 g). The head is mounted on the end of the rod. This gel contains unreacted fluids which are known to bleed through silicone rubber containers.

The resulting silicone gel filled articles were cooled, washed with isopropanol and, for purposes of this bleed evaluation study, a room temperature vulcanizable fluorosilicone sealant was spread over the patches to insure that the patches did not leak during the test period. The samples were placed in individual tissue culture dishes and evaluated visually for evidence of bleed in the manner described above. The results are tabulated in Table II. Examples 1, 4, and 5 are comparative examples.

The cumulative rating reported in Table II corresponds to the sum total of the values assigned to the degree of paper spotting observed at each interval. The numerical rating values assigned to each degree of spotting is shown at the bottom of Table II. Also taken into account were factors such as whether the spot was on the top or bottom and whether the spot covered less than the total area covered by the article. A cumulative rating value of zero indicated the least amount of bleed while higher values indicated a higher tendency to exhibit visual evidence of bleed.

The data obtained indicates that the presence of a barrier layer reduces the tendency of the articles to exhibit bleed over the period of time studied. Articles with barrier layer coatings produced from Dispersion D exhibited a greater tendency to bleed than did articles produced from Dispersion C which had a higher solids content. Among the samples prepared using Dispersion C, there did not seem to be much difference between one dip of Dispersion C versus 2 dips of Dispersion C. However, 2 dips would be preferable to insure that an adequate thickness of barrier layer coating is deposited on the inside of the container surface.

Examples 10—21

The following examples describe the preparation and evaluation of the articles of the present invention wherein the barrier layer is coated on a pre-formed silicone rubber container. The dispersion used in these Examples (Dispersion E) was prepared by blending 100 parts of a different batch of Base 2 with 10 parts of a silicone fluid slurry containing approximately 62 percent by weight of titanium dioxide pigment (this material is sold under the tradename of Ferro White GV-52 by the Ferro Corporation, Cleveland, Ohio) on a 2-roll mill. Then 10 parts of this blend was added to a jar containing 90 parts of acetone and the contents of the jar was rolled overnight to completely disperse the blend. Then 0.075 parts of Catalyst 2 and 0.04 parts of 2-methyl-3-butyn-2-ol was added to the contents of the jar and mixed well. After waiting a short time for the inhibitor to take effect, 0.2 parts of Polymer 1 was added and dispersed well to finish the preparation of Dispersion E.

Two different sizes of silicone rubber containers commonly used in the manufacture of silicone gel-filled surgically implantable mammary prostheses were obtained for use in these Examples. The containers had a wall thickness of approximately 0.25 mm and were produced by curing a platinum catalyzed dispersion similar to Dispersion A on a mandrel.

The general procedure used to coat the containers was to inject 30 cubic centimeters (cc) of Dispersion E into the Size X (225 cc volume) envelopes and 50 cc of Dispersion E into the larger Size Y (340 cc volume) containers using an 18 gauge hypodermic needle inserted directly through the container wall. Before removing the needle, a sufficient amount of air was injected into each container to remove all wrinkles. The needle was removed and a patch of unvulcanized polydimethylsiloxane gum base was placed over the needle hole. Each container was lightly powdered and placed into a gallon can (one container per can) which had holes punched in the ends to allow volatile solvent to escape. The cans were placed on a roller and the containers were allowed to tumble for a minimum of one hour at about 20 RPM. After tumbling, the coated containers were allowed to stand at room temperature for a minimum of one hour. The coatings in Example 11 and 12 were cured by heating the containers in an oven 30 minutes at 50°C and then for one hour at 175°C. The coatings in Examples 10, 16, 17 and 18 were cured by heating the containers in an oven for 15 minutes at 50° C and then for one hour at 175°C. Examples 13—15 and 19—21 are comparative Examples and did not contain a barrier layer.

Each Size X container was injected with 194 grams (330 grams for each Size Y container) of the same type of uncured silicone gel composition as was used in the previous Examples. The uncured silicone gel composition contained a mole ratio of silicon-bonded hydrogen atoms to silicon-bonded vinyl groups of 1/1.9 and when cured had a penetration value of 17.0 cm.

The bleed evaluation was done by first placing a disk of polytetrafluorethylene sheet in the bottom of a laboratory beaker, followed by four sheets of laboratory filter paper on top of the disk and finally placing one container filled with the silicone gel composition on top of the filter paper. All of the beakers containing the articles were then placed in the same oven for three and one-half hours to cure the gel composition. Upon cooling to room temperature, the silicone gel-filled article was carefully removed, the filter paper and disk weighed on an analytical balance to the nearest 0.001 gram and replaced in the beaker in the same order as done previously to insure that the same part of the article was in contact with the filter paper as before. The polytetrafluoroethylene disk is used to prevent loss of any fluid absorbed by the filter paper. The articles were then stored at room temperature and the increase in weight of the filter paper observed at 4 and 8 week intervals is tabulated in Table III. This increase in weight was attributed to bleed from the silicone gel-filled article. The articles containing a barrier layer exhibited a reduced tendency to bleed when compared to articles without such a barrier layer. It was also noted that after 8 weeks, there was no visible bleed on the surface of the articles containing a barrier layer while the comparison examples did exhibit visible bleed after the same period of time.

Example 22

This example describes an optimized formulation for use in coating pre-formed silicone rubber containers with a barrier layer. The barrier layer composition was prepared by mixing 100 parts of a gum base having the same general composition as Base 2 and 10 parts of the same type of titanium dioxide slurry used in the previous examples on a 2-roll mill. Then 80 grams of the milled mixture was cut into small pieces and added to 320 grams of dried acetone. The jar containing the acetone and milled mixture was capped and the milled mixture was completely dispersed by rolling the jar for several hours. When the contents of the jar were completely dispersed, 0.08 grams of Catalyst 1 and 0.16 grams of 1-ethynylcyclohexanol was added to the above dispersion and mixed well. Then 1.6 grams of Polymer 1 was added. The dispersion was thoroughly stirred and used.

When this dispersion is used to coat the inside of silicone rubber containers used in the production of mammary prostheses following the procedure outlined in Examples 10—12, the resulting silicone gel-filled silicone rubber articles possess a reduced tendency to exhibit surface-bleed.

Example 23

This example demonstrates the reduced tendency to exhibit surface-bleed possessed by a silicone rubber article wherein the container consists essentially of a cross-linked 3,3,3-trifluoropropylmethylpolysiloxane gum containing a silica filler. The gum base formulation used in preparing the container consisted of the following: 100 parts of a dimethylvinylsiloxy-endblocked polydiorganosiloxane gum of the same type used to prepare Base 1; 42 parts of a fume silica filler having an average pore volume of about 400 $m^2/g$; 18 parts of Fluid 1; 0.5 parts of Fluid 2 and 1 part of ammonium bicarbonate. Fluid 1 was a hydroxy-endblocked 3,3,3-trifluoropropylmethylpolysiloxane fluid having a silicon-bonded hydroxyl radical content in the range of 5.2 to 7.0 weight percent and a viscosity in the range of from 0.00007 to 0.00013 $m^2/s$. Fluid 2 was a hydroxy-endblocked polydiorganosiloxane fluid containing dimethylsiloxane units and a sufficient amount of methylvinylsiloxane units to provide from 9 to 12 weight percent of silicon-bonded vinyl radicals, said fluid containing at least 8 weight percent of silicon-bonded hydroxyl radicals and having a viscosity in the range of from 0.00002 to 0.00006 $m^2/s$.

The gum base was prepared by mixing the above gum, 13 parts of Fluid 1 and 0.25 parts of Fluid 2 in a bread dough mixer, then adding the silica filler as rapidly as possible and allowing it to become thoroughly blended. The remainder of Fluids 1 and 2 were added, the mixture was cooled and the ammonium bicarbonate was added. Mixing was continued for another 30 minutes under vacuum without any additional heating. Then, mixing was continued as the mixture was heated to 170°C and maintained at that temperature for 3 hours. It was then cooled for one hour and used.

Dispersion F was prepared by dispersing 0.5 grams (g) of zinc stearate into 100 g of the above gum base on a 2-roll mill. This material was then dispersed in 400 g of methyl isobutyl ketone. Prior to use, 3 drops (about 0.05 g) of a type Catalyst 2) see Examples 1—9) solution (this catalyst solution was neutralized with a small amount of sodium bicarbonate to remove any residual hydrogen chloride generated during manufacture) and 1.3 g of a composition consisting principally of a compound having an average formula

$$CF_3CH_2CH_2Si(OSi(CH_3)_2H)_3,$$

(a minor amount of dimeric species was also present) having a silicon-bonded hydrogen content in the range of about 0.5 to 0.7 weight percent was added to the above. A 225 cc volume container was produced by dipping a mandrel in

Dispersion F for 30 seconds, removing the mandrel and allowing the coated mandrel to air-dry for fifteen minutes. This procedure was repeated three times. After the last dip, the coated mandrel was allowed to air-dry for one hour and was then placed in an oven for four hours at 160°C.

Following the same procedures outlined in Example 1—9, the cured container was then removed from the mandrel, patched, filled with a silicone gel composition of the same type used in Examples 1—9 and the gel composition was cured. The procedure used to evaluate the tendency to exhibit bleed is described in U.S. Patent No. 4,100,627, column 6, lines 19—47 which paragraph is hereby incorporated by reference to teach that procedure. After two months storage time at room temperature, the total amount of bleed observed according to this test was 0.0594 g. Another similar article exhibited 0.1381 g of bleed after two months time although a pinhole in the container wall appeared to account for the larger amount of bleed observed in the latter. A comparable article manufactured from a silicone rubber container of the type used in Example 13 exhibited a total of 0.27 grams of bleed after two months storage at room temperature.

TABLE I

Construction of containers

| Example | Dip[a] 1 | 2 | 3 | 4 | 5 | Coating[b] | Thickness[c] |
|---|---|---|---|---|---|---|---|
| 1 | A | A | A | — | — | 0/3 | .23/.38/.30 |
| 2 | B | B | B | C | — | 1/3 | .13/.20/.15 |
| 3 | B | B | B | C | C | 2/3 | .15/.30/.23 |
| 4 | B | B | B | — | — | 0/3 | .15/.23/.20 |
| 5 | B | B | B | B | — | 0/4 | .22/.22/.22 |
| 6 | B | B | B | C | — | 1/3 | .17/.18/.18 |
| 7 | B | B | B | D | — | 1/3 | .18/.20/.20 |
| 8 | B | B | B | C | C | 2/3 | .18/.20/.20 |
| 9 | B | B | B | B | D | 1/4 | .23/.23/.23 |

(a) The letters refer to the dispersions used.
(b) The first figure is the number of dips of fluorine-containing organopolysiloxane dispersion and the second figure is the number of dips of polydimethylsiloxane dispersion.
(c) Top/Bottom/Average container wall thickness in mm.

TABLE II
Visual evaluation of tendency to bleed

| Example | Observation time[a] 1 Day | 2 Days | 7 Days | 15 Days | 32 Days | 8 Weeks | Cumulative rating | Coating[b] |
|---|---|---|---|---|---|---|---|---|
| 1 | NS | NS* | MS | MS,SG | MS,HG | MS,HG | 26 | 0/3 |
| 2 | NS | NS | NS | NS | VSS(TO),D | VSS(TO),D | 2 | 1/3 |
| 3 | NS | NS | VSS(BO) | VSS(BO) | VSS(BO),D | VSS(BO),D | 4 | 2/3 |
| 4 | NS* | NS* | MS | HS,SG | HS,HG | HS,HG | 30 | 0/3 |
| 5 | NS* | NS* | MS,SG | HS,SG | HS,HG | HS,HG | 30 | 0/4 |
| 6 | NS | NS | NS | VSS(BO) | MS(BT),D | VSS(TO)—MS(BO),D | 7 | 1/3 |
| 7 | NS** | NS*,** | VSS** | SS | MS,SG | MS,VSG** | 16 | 1/3 |
| 8 | NS | NS | NS | NS | NS | NS,D | 0 | 2/3 |
| 9 | NS** | NS*** | MS** | MS | MS,HG | HS,HG | 26 | 1/4 |

[a] First Character: Paper Spot (Rating Value): NS—No Spot (0); VSS—Very Slight Spot (2); SS—Slight Spot (4); MS—Medium Spot (6); HS—Heavy Spot (8). The letters in parentheses indicate whether the Top Only (TO), Bottom Only (BO) or the Bottom and Top (BT) showed spots.
Second Character: Container Greasiness to Touch: D—Dry; SG—Slightly Greasy; MG—Medium Greasy; HG—Heavy Grease (visible fluid on surface)
White filter paper—spot visible when held up to light.
** Bumpy irregular surface—rating of slight—may be due to envelope swelling caused by bleed
*** Bumpy irregular surface—rating of medium—may be due to envelope swelling caused by bleed

[b] From Table I.

TABLE III
Pre-formed container bleed evaluation

| Example | Container size | Barrier layer | Increase in weight of filter paper (grams) 4 weeks | 8 weeks |
|---|---|---|---|---|
| 10 | X | YES | 0.039 | 0.039 |
| 11 | X | YES | 0.021 | 0.028 |
| 12 | X | YES | 0.016 | 0.030 |
| 13 | X | NO | 0.192 | 0.165 |
| 14 | X | NO | 0.158 | 0.199 |
| 15 | X | NO | 0.185 | 0.213 |
| 16 | Y | YES | 0.035 | 0.028 |
| 17 | Y | YES | 0.039 | 0.035 |
| 18 | Y | YES | 0.026 | 0.036 |
| 19 | Y | NO | 0.239 | 0.292 |
| 20 | Y | NO | 0.260 | 0.347 |
| 21 | Y | NO | 0.359 | 0.398 |

## Claims

1. A silicone gel-filled silicone rubber article possessing a reduced tendency to exhibit surface-bleed comprising

(A) a flexible container (21) of silicone rubber consisting essentially of a cross-linked gum of polydimethylsiloxane, said container being filled with

(B) a silicone gel composition (23) consisting essentially of a silicone gel of cross-linked polydimethylsiloxane, characterized in that there is

(C) an essentially continuous barrier layer (22) of a composition consisting essentially of fluorine-containing organopolysiloxane situated between the interior of container (A) and the gel composition of (B), said barrier layer being coated on the interior wall of container (A).

2. The article in accordance with claim 1 wherein the fluorine-containing organopolysiloxane of (C) is 3,3,3-trifluoropropylmethylpolysiloxane.

3. The article in accordance with claim 2 wherein said fluorine-containing organopolysiloxane is a cross-linked gum.

4. The article in accordance with claims 1 to 3 which is an external mammary prosthesis.

5. The article in accordance with claims 1 to 3 which is a surgially implantable mammary prosthesis.

6. The article in accordance with claim 4 which contains pigments simulating skin color.

7. A silicone gel-filled silicone rubber article possessing a reduced tendency to exhibit surface-bleed characterized in that it comprises:

(A) a flexible container of fluorosilicone rubber consisting essentially of a cross-linked gum of fluoroalkylpolysiloxane filled with

(B) a silicone gel composition consisting essentially of a silicone gel of cross-linked polydimethylsiloxane.

8. The article in accordance with claim 7 wherein the fluoroalkylpolysiloxane of (A) is 3,3,3-trifluoroprcpylmethylpolysiloxane.

9. The article in accordance with claim 8 which is an external mammary prosthesis.

10. The article in accordance with claim 9 which contains pigments simulating skin color.

## Revendications

1. Un article de caoutchouc de silicone rempli de gel de silicone ayant une tendance réduite à présenter de une exsudation en surface, qui comprend

A) un récipient flexible (21) en caoutchouc de silicone constitué essentiellement d'une gomme réticulé polydiméthylsiloxane, ce récipient étant rempli de

B) une composition de gel de silicone (23) constitué essentiellement d'un gel de silicone de polydiméthylsiloxane réticulé, caractérisé en ce qu'il comprend en outre,

C) une couche essentiellement continue formant barrière (22) constituée essentiellement d'un organopolysiloxane contenant du fluor, disposée entre l'intérieur du récipient (A) et la composition de gel (B), cette couche-barrière étant appliquée en un revêtement sur la paroi intérieure du récipient (A).

2. Un article selon la revendication 1, dans lequel l'organopolysiloxane contenant du fluor de (C) et du 3,3,3-trifluoropropylméthylpolysiloxane.

3. Un article selon la revendication 2, dans lequel l'organopolysiloxane contenant du fluor est une gomme rèticulée.

4. Un article selon les revendications 1 à 3, qui est une prothèse mammaire externe.

5. Un article selon les revendications 1 à 3, qui est une prothèse mammaire chirurgicalement implantable.

6. Un article selon la revendication 4, qui contient des pigments simulant la couleur de la peau.

7. Un article de caoutchouc de silicone rempli de gel de silicone aynt une tendance réduite à présenter une exsudation en surface, caractérisé en ce qu'il comprend:

A) un récipient flexible de caoutchouc de fluorosilicone constitué essentiellement d'une gomme réticulée de fluoroalcoylpolysiloxane, rempli de
B) une composition de gel de silicone constituée essentiellement d'un gel de silicone de polydiméthylsiloxane réticulé.

8. Un article selon la revendication 7, dans lequel le fluoroalcoylpolysiloxane de (A) est du 3,3,3-trifluoropropylméthylpolysiloxane.

9. Un article selon la revendication 8, qui est une prothèse mammaire externe.

10. Un article selon la revendication 9, qui contient des pigments simulant la couleur de la peau.

**Patentansprüche**

1. Mit einem Silicongel gefüllter Silicongummigegenstand mit erniedrigter Neigung zum Austreten des Gels auf der Oberfläche des Gegenstands enthaltend

(A) einen flexiblen Behälter (21) aus Silicongummi bestehend im wesentlichen aus einem vernetzten gummiartigen Harz aus Polydimethylsiloxan, wobei dieser Behälter gefüllt ist mit
(B) einer Silicongelzusammensetzung (23) bestehend im wesentlichen aus einem Silicongel aus vernetztem Polydimethylsiloxan, dadurch gekennzeichnet, daß
(C) eine im wesentlichen kontinuierliche Sperrschicht (22) bestehend im wesentlichen aus einem fluorhaltigen Organopolysiloxan zwischen dem Inneren des Behälters (A) und der Gelzusammensetzung (B) vorhanden ist, wobei die Sperrschicht einen Überzug auf der Innenwand des Behälters (A) bildet.

2. Gegenstand gemäß Anspruch 1, dadurch gekennzeichnet, daß das fluorhaltige Organopolysiloxan (C) 3,3,3-Trifluorpropylmethylpolysiloxan ist.

3. Gegenstand gemäß Anspruch 2, dadurch gekennzeichnet, daß das fluorhaltige Organopolysiloxan ein vernetztes Gummi ist.

4. Gegenstand gemäß den Ansprüchen 1 bis 3, dadurch gekennzeichnet, daß er eine äußere Brustprothese ist.

5. Gegenstand gemäß den Ansprüchen 1 bis 3, dadurch gekennzeichnet, daß er eine chirurgisch einpflanzbare Brustprothese ist.

6. Gegenstand gemäß Anspruch 4, dadurch gekennzeichnet, daß er Pigmente enthält, die die Hautfarbe simulieren.

7. Mit einem Silicongel gefüllter Silicongummigegenstand mit erniedrigter Neigung zum Austreten des Gels auf der Oberfläche des Gegenstandes, dadurch gekennzeichnet, daß er enthält

(A) einen flexiblen Behälter aus einem Fluorsiliconkautschuk, der im wesentlichen aus einem vernetzten Gummi von Fluoralkylpolysiloxan gefüllt mit
(B) einer Silicongelzusammensetzung, bestehend im wesentlichen aus einem Silicongel eines vernetzten Polydimethylsiloxans.

8. Gegenstand gemäß Anspruch 7, dadurch gekennzeichnet, daß das Fluoralkylpolysiloxan (A) 3,3,3-Trifluorpropylmethylpolysiloxan ist.

9. Gegenstand gemäß Anspruch 8, dadurch gekennzeichnet, daß er eine äußere Brustprothese ist.

10. Gegenstand gemäß Anspruch 9, dadurch gekennzeichnet, daß er Pigmente enthält, die die Hautfarbe simulieren.

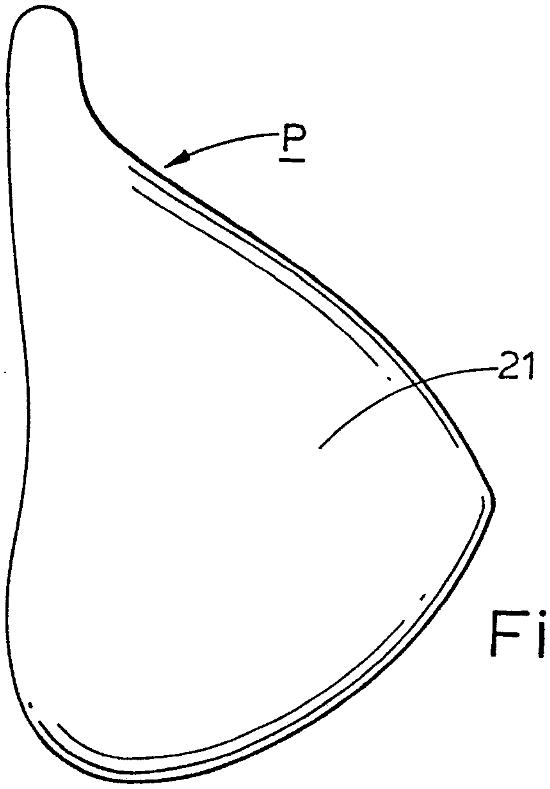


Fig.1

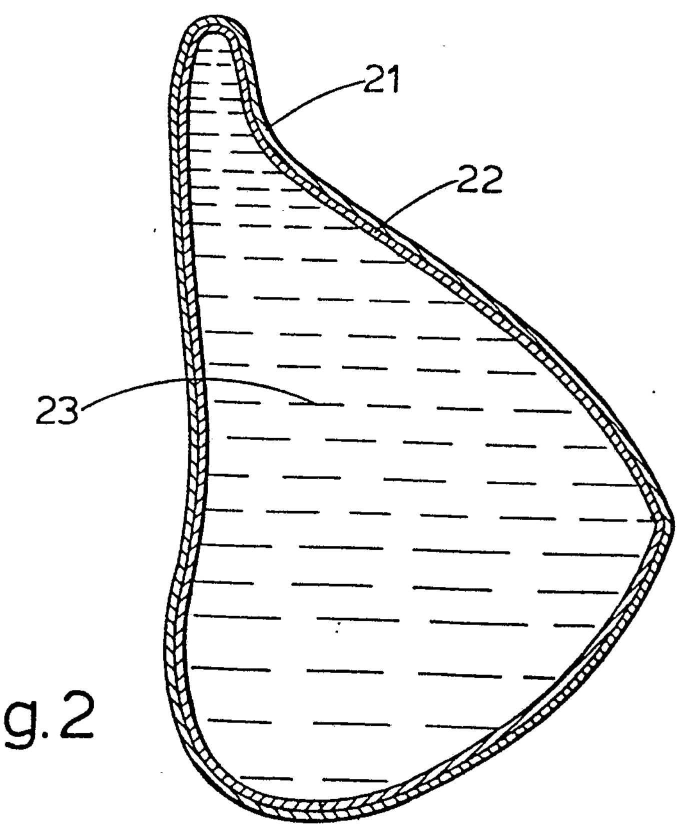


Fig.2